# EUROPEAN PATENT SPECIFICATION

(11) **EP 1 937 171 B1**
(45) Date of publication and mention of the grant of the patent: **17.03.2010**
(21) Application number: 06803635.9
(22) Date of filing: 15.09.2006
(51) Int. Cl.: A61B 17/72, A61B 17/80

(54) **INTRAMEDULLARY BONE PLATE WITH SHEATH**
INTRAMEDULLÄRE KNOCHENPLATTE MIT HÜLSE
PLAQUE OSSEUSE INTRAMÉDULLAIRE À GAINE

(30) Priority: 20.09.2005 US 231710
(43) Date of publication of application: 02.07.2008
(60) Divisional application: 10152179.7
(73) Proprietor: DVO Extremity Solutions, LLC, Warsaw, Indiana 46580 (US)
(72) Inventor: RUNNING, Donald Eli, Warsaw, Indiana 46580 (US); ONDRLA, Jeffrey Michael, Leesburg, Indiana 46538 (US); HUNT, III, M.D., Thomas R., Birmingham, Alabama 35223 (US); CHURCHILL, M.D., R. Sean, Mequon, Wisconsin 53092 (US)
(74) Representative: Thomson, Craig Richard
(86) International application number: PCT/US2006/035920
(87) International publication number: WO 2007/035440

(56) References cited:
- WO-A-03/101320
- US-A- 5 603 715
- US-A1- 2004 210 217
- US-B1- 6 358 250

## Description

### Field of the Invention

This invention relates generally to implantable, surgical devices for implantation and, in particular, to an improved surgical device to be used in the internal fixation of fractures to the distal radius and other long bones.

### Background of Invention

There are a variety of surgical devices and methods that are being used to treat fractures of the distal radius. Historically, open reduction and internal fixation (ORIF) of distal radius fractures has been accomplished by the implantation of various types of metallic plates, pegs, wires and screws utilizing a dorsal approach and securing such plates on the dorsal aspect of the radius. Examples of such devices can be found in U.S. Patent Nos. 6,706,046 and 6,730,090. Implanting surgical devices using a dorsal approach is technically easier as critical vascular and soft tissue structures are avoided. A shortcoming and surgical complication caused by the prior art and the corresponding dorsal approach is an increased potential for tendonitis and/or tendon rupture caused by the device thickness and non-uniformity of the adj acent device surface. The prior art also has not been designed to stabilize comminuted fractures of the distal radius in that these devices medial-lateral width is too narrow to adequately span and immobilize fracture sites. Further, the prior arts' use of fixation pegs with set angle orientations does not allow for adequate bone fragment fixation in distal radius fractures.

The state of the art for treating fractures of the distal radius has recently shifted to ORIF utilizing a volar approach. The reason for the shift was the possible elimination of tendonitis and/or tendon rupture that had been experienced with surgical devices implanted dorsally. The shortcoming of the volar approach is the presence of significant soft tissue and vascular anatomy and the resulting technically challenging

implantation procedure of the surgical device. Examples of surgical devices implanted using a volar approach include U.S. Patents Nos.: 6,440,135,6,364,882, 6,508,819, 6,358,250, 6,893,444, 6,767,351 and 6,712,820. Another example of a surgical device for bone fragment fixation is US 2004/0210217 which describes a bone plate for use in interior lumber spinal fixation. It has interlocking components to prevent dislodgement of the plate due to anatomical forces. The exposed surface of the plate is smooth to prevent trauma to internal body tissue. The plate spans the intervertebral space with each end attached to an adjacent vertebrae by locking screws threadably engaged with tubular bone anchors. The bone anchors are threaded into the vertebrae and have a reduced diameter leading end which the locking screws expand to lock the anchor and the vertebrae and secure the end of the locking screw and the anchor. The plate has a counter-sunk cavity including portions overlapping the locking screws. The locking cap fits in the cavity to secure the other end of the locking screws from backing out of the plate The invention described herein addresses these and other shortcomings of the prior art.

### Summary of the Invention

The present invention provides an intramedullary bone plate with sheath having an intramedullary stem element that is composed of a proximal portion and a distal portion. The exterior surface of the distal portion being configured, for example shaped or dimensioned by machined means, surface treatments or applied three-dimensional surface coatings to enhance bone fixation and rotational stability. The proximal portion being of a smaller circular diameter and having an arced geometry in the sagittal plane, allowing for intramedullary contact and securement.

In another aspect, the bone plate of the present invention also includes a bone plate head element with the medial side of the bone plate head consisting of a downward angled and outward projecting tab member. This medial tab member sits over the medial side of the radius when the intramedullary stem is properly inserted. Numerous angled, non-threaded and through bone screw and surgical k-wire holes may be located in the bone plate head allowing for fixation of bone fragments and anatomic reconstruction of the fractured distal radius. All bone screw holes are typically located in the lateral and central aspects of the bone plate head, with all surgical k-wire holes preferably being located in the medial tab member. A threaded screw hole with a centerline perpendicular to the top surface of the bone plate head may be located in the sheath recess of the bone plate head.

A neck element rigidly connects the bone plate head to the intramedullary stem. The neck element originates at the most distal end of the intramedullary stem and angles in an upward direction connecting to the most proximal edge or bottom of the bone plate head. The neck element offsets axially and longitudinally in the sagittal plane, the intramedullary stem from the bone plate head.

In yet another aspect of the present invention, a sheath element may be attached to the bone plate head. The sheath fits within the sheath recess and may be secured by the sheath screw that engages both the sheath and bone plate head. When in the recess, the sheath covers all of the bone screw heads. The sheath may also include nobs or recesses located on the bottom surface wherein when inserted, the nobs would typically project onto the opposing bone screw head wherein the recesses would receive the bone screw head. These nobs and recesses are intended to substantially inhibit any movement by the screw from its implanted position. The sheath, when joined with the bone plate head, is preferably of minimal overall thickness, thereby giving the invention a low profile and congruent surface for the adjacent contacting soft tissue.

The present invention is used for treating distal radius fractures and fractures of similar types in other long bones. Typically the intramedullary stem is inserted into the medullary canal of the bone through the fracture site. The intramedullary stem is then seated and the bone plate head is aligned over the fracture site while ensuring the medial tab member is located over the medial bone fragment. By aligning and seating the bone plate head, the fracture is reduced and buttressed. While maintaining the set position of the fracture, holes may be drilled through the bone plate head into the bone fracture fragments. The drill holes and inserted bone screws are typically at set angles as determined by the bone plate head. Following placement of the bone screws, the sheath may be set into the sheath recess with the bottom surface nobs preferably making contact with the two distal bone screw heads or in the alternative, the bone screw heads preferably projecting into the recesses. The sheath screw typically engages both the sheath and bone plate head and draws the sheath tightly into the sheath recess. Lastly, surgical k-wire may be inserted through the holes located in the medial tab member. The surgical k-wire would be used to secure any bone fracture fragments situated near the medial side of the bone. Following final placement of the surgical k-wire, the free ends may be cut and bent into the wire channel that longitudinal connects the holes located on the top surface of the medial tab member.

### Brief Description of the Drawings

The subject matter which is regarded as the invention is particularly pointed out and distinctly claimed in the claims at the conclusion of the specification. The features and advantages of the invention will be apparent from the following detailed description taken in conjunction with the accompanying drawings, which drawings illustrate several embodiments of the invention.

FIG. 1 is a distal end top perspective view of the subject invention.

FIG. 2 is a top view of the subject invention.

FIG. 3 is a lateral view of the subject invention.

FIG. 4 is a cross-section view of the bone plate head element along line 4-4.

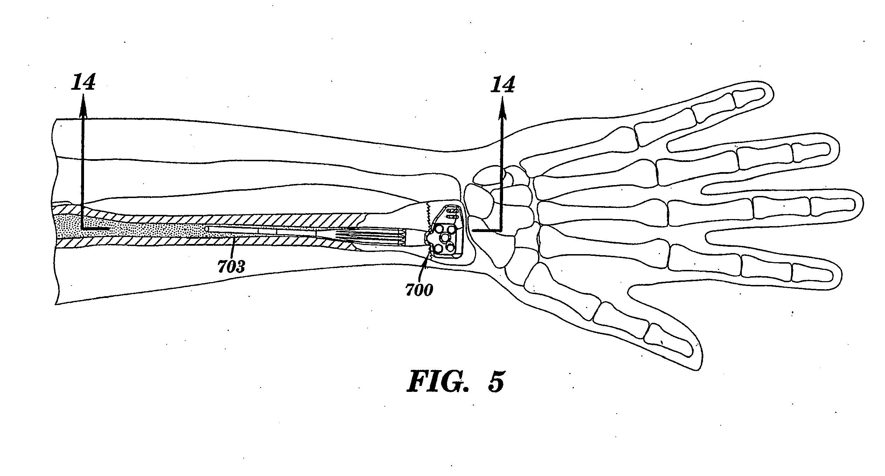

FIG. 5 is a top view of the radius and ulna with the implanted subject invention.

FIG. 6 is a cross-section of the distal portion of the intramedullary stem element with the longitudinal running flutes along line 6-6.

FIG. 7 is a distal end top perspective view of the sheath element.

FIG. 8 is a proximal end bottom perspective view of the sheath element.

FIG. 9 is a transverse plane section view of the distal-medial and distal-lateral bone screw holes.

FIG. 10 is a sagittal plane section view of the distal-medial and distal-lateral bone screw holes.

FIG. 11 is a side view of the bone screw.

FIG. 12 is a side view of the sheath screw.

FIG. 13 is a transverse plane section view of the proximal-lateral bone screw hole.

FIG. 14 is a side view of the implanted invention.

FIGS. 15-18 show the method used in treating distal radius fractures with the invention.

FIG. 19 is an alternative embodiment of the invention with a surface coating applied to the intramedullary stem.

FIG. 20 is an alternative embodiment of the invention with porous coating applied to the intramedullary stem element.

FIG. 21 is an alternative embodiment of the invention with a straight proximal portion to the intramedullary stem element.

FIG. 22 is an alternative embodiment of the invention showing an exploded view of the modular bone plate head element and neck element.

FIG. 23 is an alternative embodiment of the invention showing an assembly view of the modular bone plate head element and neck element.

FIG. 24 is an alternative embodiment of the invention showing the bone plate head element with the proximal edge hood.

FIG. 25 is an alternative embodiment of the invention showing an assembly view of the straight proximal portion to the intramedullary stem element and the bone plate head element fixed with a neck element angle of ninety degrees.

FIG. 26 is a proximal and bottom perspective of an alternative embodiment of the sheath.

### Detailed Description of the Invention

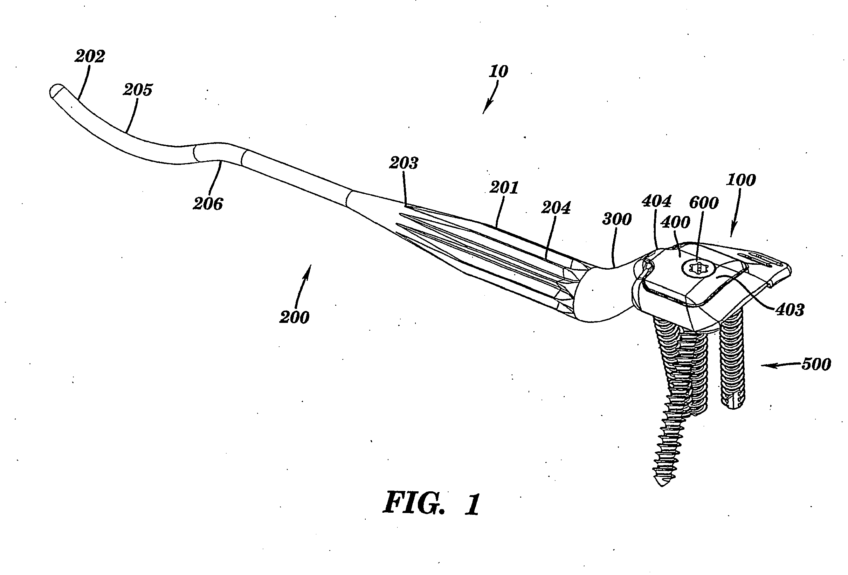

FIG. 1 shows the general arrangement of a preferred embodiment of the intramedullary bone plate with sheath 10 of this invention. Generally, the intramedullary bone plate with sheath 10 includes a bone plate head 100, an intramedullary stem 200, a connecting neck 300, a sheath 400, a bone screw 500, a sheath screw 600 and surgical k-wire (not shown). The various embodiments of the present invention, as described in greater detail below, result in the intramedullary bone plate with sheath designed to allow for greater intraoperative flexibility and fracture stabilization.

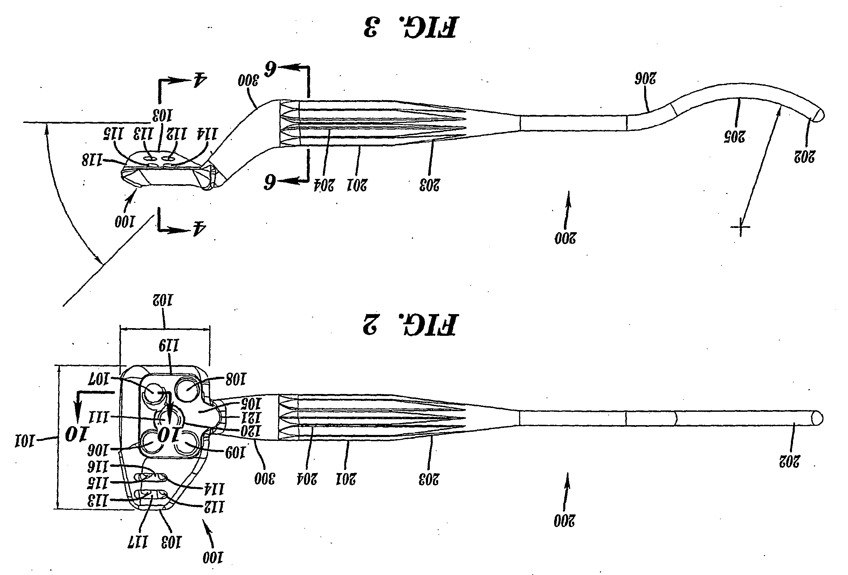

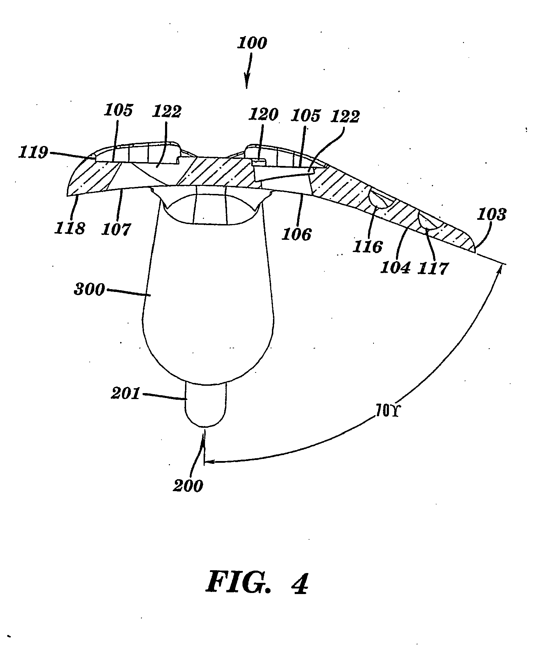

With reference to FIG. 2, the bone plate head 100 is shaped with the overall head width 101 being greater than or equal to one half that of the longitudinal length of the head 102. As shown in FIG. 4, the medial tab member 103 is directed downward at an angle of approximately seventy degrees relative to the transverse plane, allowing for increased bone fragment capture. A sheath recess 105 may be oriented in the central aspect of the bone plate head 100 allowing for the insertion of the sheath 400. FIG. 4 also shows the sheath recess 105 including at least one raised boundary 119 on the lateral side of the bone plate head 100. As shown in FIG. 2, in the preferred embodiment, the boundary 119 continues around to the proximal side of the bone plate head 100 forming a tab shaped alcove 121 at the neck 300 bone plate head 100 junction. FIGS. 2 and 4 also show a raised circular collar 120 being approximately concentric with the threaded screw hole 111. In the embodiment shown in FIG. 2, three counter-bored non-threaded bone screw holes 106, 107, 109 are located in the central aspect of the bone plate head 100.

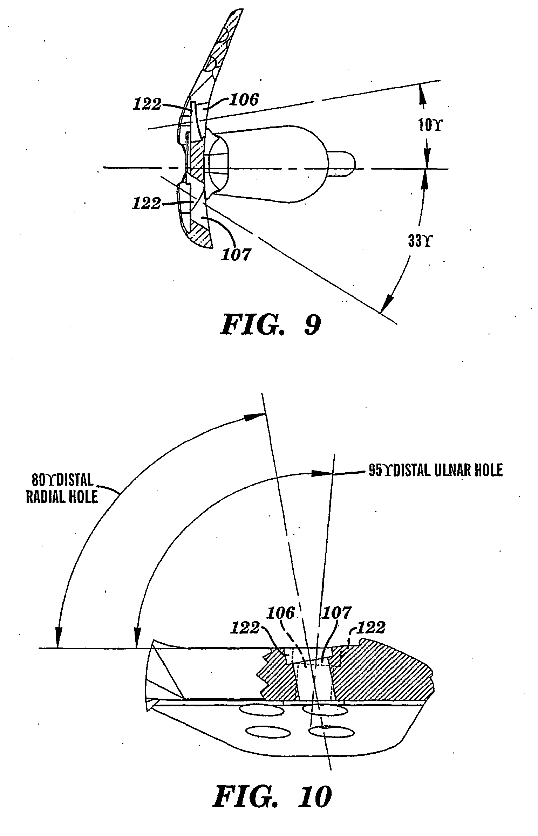

As shown in FIG. 9, the distal-medial screw hole 106 allows for preferably, for example, ten degrees of outward angulation relative to the centerline line of the intramedullary stem 200 and as shown in FIG. 10, the screw hole 106 allows for, preferably, for example, ninety-five degrees relative to the top surface of the bone plate head 100. As shown in FIG. 9, the distal-lateral screw hole 107 allows for, preferably, for example, thirty-three degrees of outward angulation relative to the centerline line of the intramedullary stem 200 and as shown in FIG. 10, the screw hole 107 allows for, preferably, for example, eighty degrees relative to the top surface of the bone plate head 100. The centerline of the proximal-medial screw hole 109 is substantially parallel to the centerline line of the intramedullary stem 200 and about normal to the top surface of the bone plate head 100.

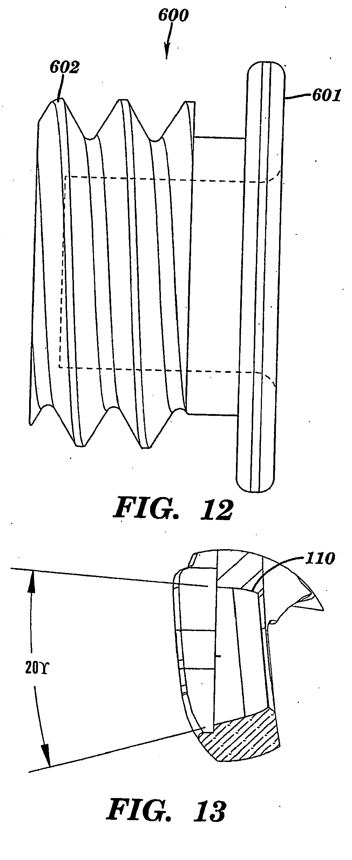

As seen in FIG. 13, the proximal-lateral screw hole 108 may include a spherical seat area 110 allowing for the bone screw 500 to pivot in an inward and outward direction with a preferable overall range of twenty degrees relative to the centerline of the intramedullary stem 200 and a preferable overall range of twenty degrees in the proximal-distal direction. Referring again to FIG. 2, a threaded through hole 111 is preferably located in the central aspect of the sheath recess 105. The centerline of the threaded hole 111 is oriented about normal to the top surface of the sheath recess 105. The sheath screw 600 may be threaded into the threaded hole 111 as described below, following the placement of the sheath 400 into the sheath recess 105.

As shown in FIG. 2 , the medial tab member 103 may include, for example, through holes 112, 113, 114, 115 of smaller diameter relative to bone screw holes 106, 107, 108, 109. Each of the four holes 112, 113, 114, 115 are preferably angled forty-five degrees proximally and fifteen degrees laterally. The medial-distal and medial-proximal holes 114, 115 may be connected by a longitudinal channel 116 and the lateral-distal and lateral-proximal holes 112, 113 may also be connected by a longitudinal channel 117. These longitudinal channels allow for the insertion of surgical k-wire therethrough. FIG. 4 shows the longitudinal channels 116, 117 running substantially parallel to each other and to the centerline of the intramedullary stem 200. Following the insertion of surgical k-wires and securement of bone fragments with these wires, the free ends may pass through bottom surface 104 of the medial tab member 104 and through any of the four holes 112, 113, 114, 115. Dependent upon the exiting hole 112, 113, 114, 115, the free end of the wire may be bent into the adjacent channel 116, 117, with the free end typically being inserted into the corresponding connected hole 112, 113, 114, 115. As illustrated in FIG. 4, the bottom surface of the bone plate head 118 is relatively concave allowing for increased bone-bone plate head contact, while the bottom surface of the medial tab member 104 is typically constructed with a flat geometry.

Referring to FIGS. 1, 2 and 3, the intramedullary stem 200 is comprised of a distal portion 201, a proximal portion 202 and a mid-shaft portion 203. The distal portion 201 is preferably a circular cross-section that then tapers to approximately match and connect to the smaller circular cross-section of the proximal portion 202. FIGS. 1, 2 and 3 show the taper member being located approximately in the mid-shaft portion 203. The taper member usually extends through the mid-shaft portion 203 until it matches the smaller circular diameter of the proximal portion 202.

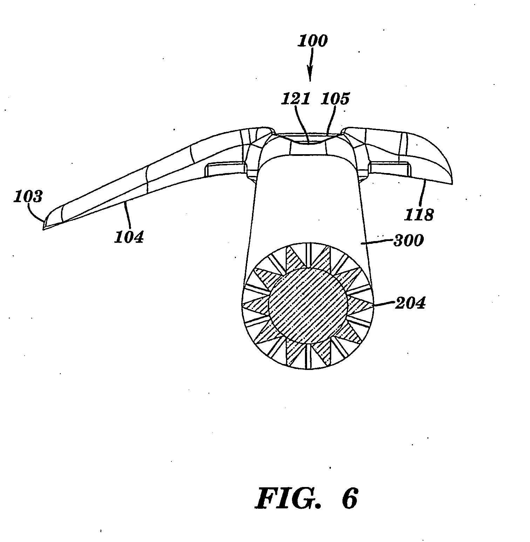

FIG. 6 shows the longitudinally running flutes 204 that may be machined into the exterior circumference surface of the distal portion 201. The flutes 204 are shaped and dimensioned preferably for the purpose of medullary canal fixation within the bone and rotational control of the invention 10. As shown in FIGS. 1, 2 and 3, the flutes 204 extend from the most distal end of the distal portion 201 to approximately the mid-point of the taper member in the mid-shaft portion 203.

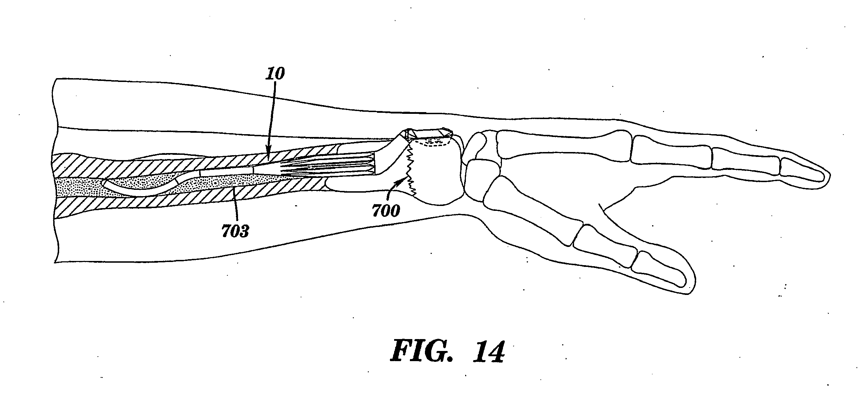

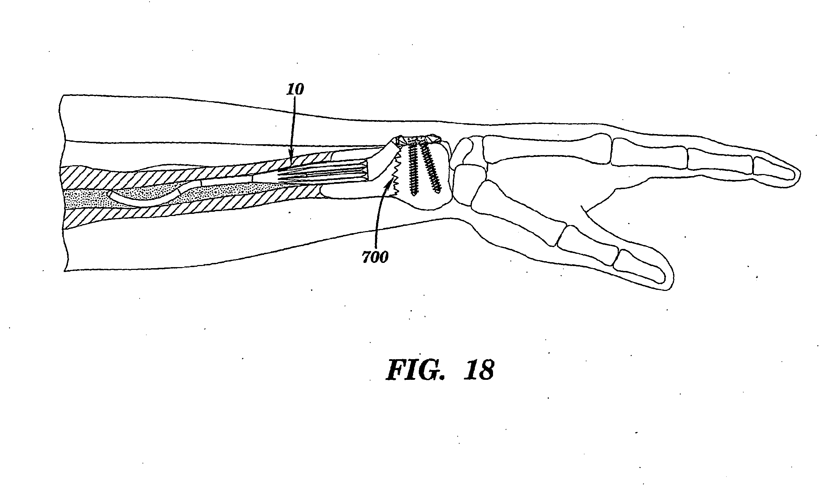

With reference to FIG. 2, the proximal portion 202 is relatively straight with respect to the coronal plane. FIGS. 2 and 3 show the proximal portion 202 being preferably bi-arced with respect to the sagittal plane. As seen again in FIG. 3, the downward projecting arc 205 runs from about the proximal tip of the intramedullary stem 200 to approximately the mid-point of the proximal portion 202. An upward projecting arc 206 of a slightly lesser radius starts approximately at the distal end of the downward arc 205 and extends to about proximal end of the mid-shaft portion 203. As shown in FIGS. 14 and 18, the combination of these two opposing arcs allows for three-point fixation within the bone's medullary canal by the intramedullary stem 200.

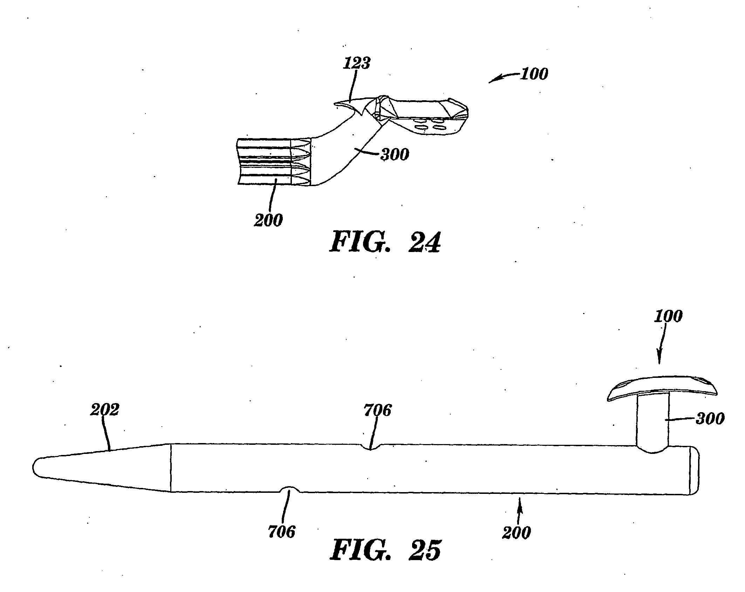

FIG. 14 further shows that implant fixation and stability may be achieved through multiple mechanisms, including but not limited to, the three point fixation provided by the intramedullary stem 200, the interference fit of the flutes 204, or a combination of these two mechanisms. Additionally, axial implant fixation and stability is partially achieved by the proximal side of the bone plate head 100 abutting the cortical wall of the distal radius. FIG. 24 shows an alternative embodiment of the invention 10, wherein the entire proximal side of the bone plate head 100 has a distinct hood 123 that projects in the proximal direction making intimate contact with the cortical wall of the distal radius.

FIG. 3 shows the connecting neck element 300 between the most distal end of the intramedullary stem 200 and typically, the proximal edge of the bone plate head 100. Referring again to FIG 3, the neck 300 essentially offsets the intramedullary stem 200 and the bone plate head 100 in two directions. In the sagittal plane, the centerline of the intramedullary stem 200 is substantially parallel to the centerline of the bone plate head 100, while in the coronal and sagittal planes the intramedullary stem 200 is longitudinally offset from the bone plate head 100. As seen in FIG. 3, the neck 300 is typically set at an acute angle relative to the centerline of the intramedullary stem 200, although this angle may reach ninety degrees. With reference to FIGS. 2 and 3, the neck 300 may have a slight reverse taper in that the cross-section of the neck 300 at the bone plate head junction may be smaller relative to the cross-section of the neck 300 at the intramedullary stem junction.

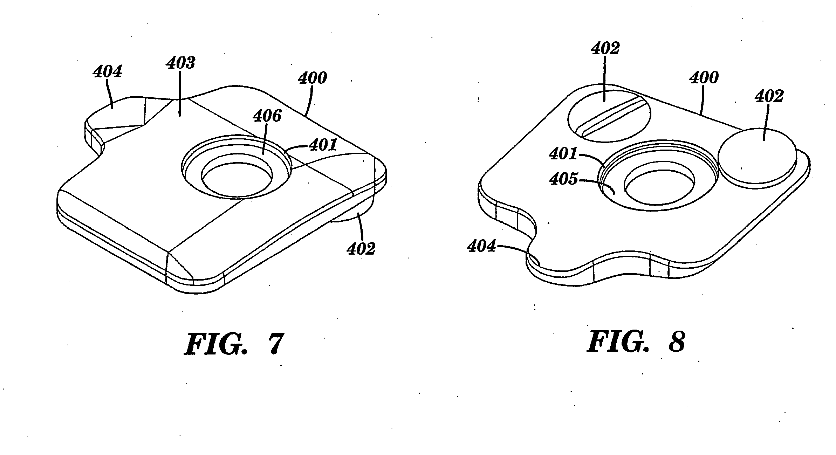

As seen in FIG. 1, the sheath 400 typically has a smooth convex top surface 403 that when inserted into the sheath recess 105 is usually contiguous with the outer aspects of the bone plate head 100. When joined, the bone plate head 100 and the sheath 400 preferably form a low-profile and congruent surface that will allow for non-disruption of the dissected soft-tissue. As seen in FIG. 7, the sheath 400 is essentially a rectangular shape with a tab component 404 extending from the proximal side that may insert into the alcove 121. A threaded through hole 401 is typically located slightly off-center from both the medial-lateral and proximal-distal direction. As shown in FIG. 7, the threaded hole 401 may have a counter-bore 406 to allow for the sheath screw head 601 to sit flush with the top surface 403 when fully engaged. FIG. 1 shows the sheath screw 600 fully inserted. FIG. 8 illustrates the bottom surface of the sheath 400, the nobs 402 and the circular groove 405 that is approximately concentric to the threaded hole 401. Referring again to FIG. 8, the nobs 402 typically are cylinder-like members preferably projecting from the bottom surface of the sheath 400. When the sheath 400 is inserted into the sheath recess 105, the irregular ends of the nobs 402 may project onto the heads of the inserted distal-lateral bone screw 500 and distal-medial bone screw 500 preferably substantially inhibiting any movement of these bone screws 500 from their implanted positions as seen in FIG. 1. An alternative embodiment of the sheath 400 comprises recesses 407 preferably located on the bottom surface of the sheath 400. In this embodiment, when the sheath 400 is inserted into the sheath recess 105, the recesses 407 align with the dome shaped heads (not shown) of the inserted bone screws 500, preferably substantially inhibiting any movement of the bone screws 500 from their implanted positions.

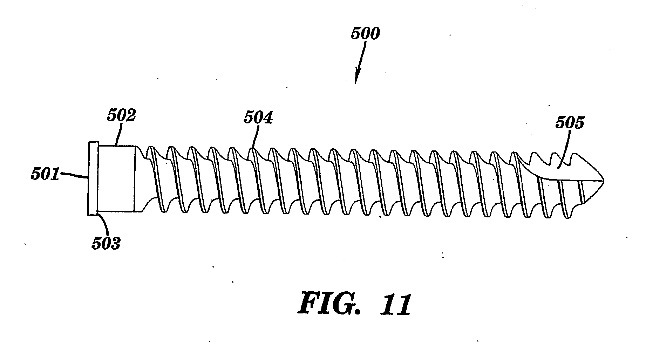

The bone screw 500 as seen in FIG. 11 may be used in conjunction with the bone plate head 100 to secure bone fragments and reduce the distal radial fracture 700. The bone screw 500 is typically available in various lengths. The length of bone screw 500 utilized is usually dependent upon the size and orientation of the bone fragment. The screw head 501 typically has a star shaped indention on the top that matches the insertion tool head (not shown) and is flat. In an alternative embodiment of the bone screw 500, the screw head 501 would be dome shaped (not shown). The screw head 501 has a slight undercut that then transitions into the screw shank 502. The screw shank 502 preferably has a diameter equal to the major diameter of the bone screw 500. The undersurface of the screw head 503 is relatively flat thereby allowing the bone screw 500 to comfortably sit within the counterbore 122 of the bone screw holes 106, 107,109. The threads 504 are machined to allow the bone screw 500 to self-tap.

As seen in FIG. 12, the sheath screw 600 has a predominately flat head 601 and typically has the same a star shaped invention as the bone screw 500. The flat head 601 typically allows for the sheath screw 600, when fully inserted, to sit flush with the sheath top surface 403. The sheath screw 600 when threaded engages both the sheath 400 and the bone plate head 100. Alternatively, the sheath 400 and the bone plate head 100 may be assembled and secured by means other than the sheath screw 600. These other means include but are not limited to, multiple sheath screws, a hinge element fixing the sheath 400 and bone plate head 100 on one side with a snap-like locking element or screw on the opposing side of the hinge element, or a snap-like locking elements on opposing sides of the sheath 400 that may lock within a corresponding opening on the bone plate head 100.

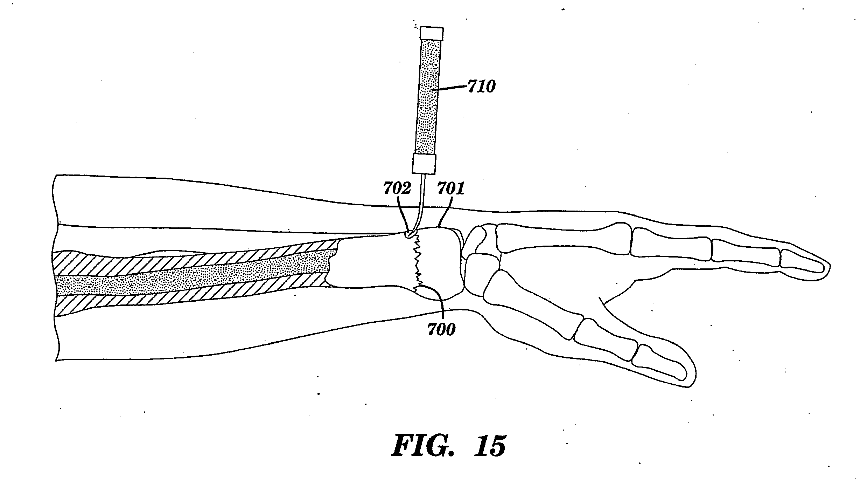

The preferred embodiment of the invention 10 may be used to treat distal fractures of the radius 700 and other similar types of fractures in long bones. For distal radius fractures, typically, the implantation method commences with a skin incision being made on the dorsal aspect of the distal radius that is over the 3^{rd} extensor compartment. Several soft tissue structures, including the extensor tendons may be dissected and distracted from the site, with heightened care being taken to protect the radial sensory nerve. The fracture 700 and the involved distal radius are exposed. As shown in FIG. 15, if Lister's tubercule 701 is not already fragmented, a rongeur or other cutting device (not shown) is used to remove the prominence. If access to the medullary canal is not obvious, then an awl 710 is used to prepare an initial opening.

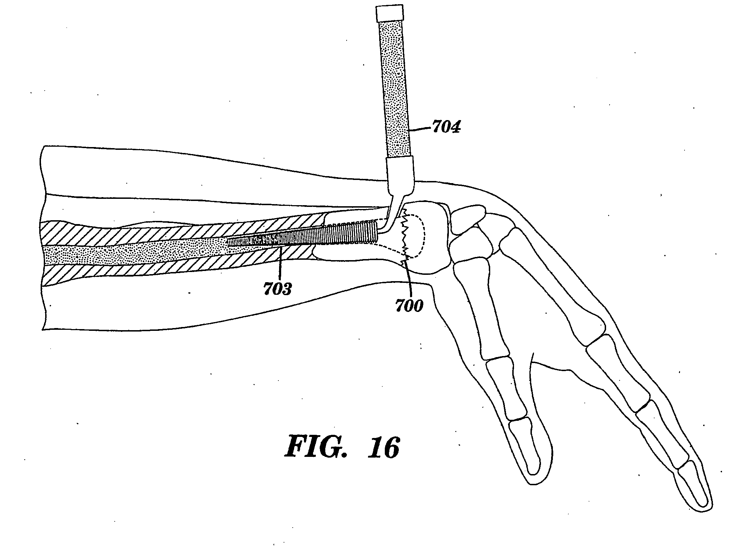

In further preparation of the implant site and the medullary canal 703, FIG. 16 illustrates the use of the one-piece broach 704 that may be inserted into the medullary canal 703 while the wrist is in a relatively flexed position. In the event further seating of the broach is desired, a twist drill may be used to notch the dorsum (not shown).

The intramedullary stem 200 may encounter mild resistance when inserted as it makes contact with the walls of the medullary canal 703. If the distal aspect of the bone plate head 100 overhangs the radiocarpal joint, a notch may be made with a twist drill or rongeur (not shown) in the distal radius allowing the neck 300 to seat further proximally. Once properly placed, the bone plate head 100 will typically be directly under the previous dissected EPL and EDC tendons (not shown) and as a result of the buttressing effect of the seating process and bone plate head 100 placement, the alignment of the fracture 700 should be markedly improved.

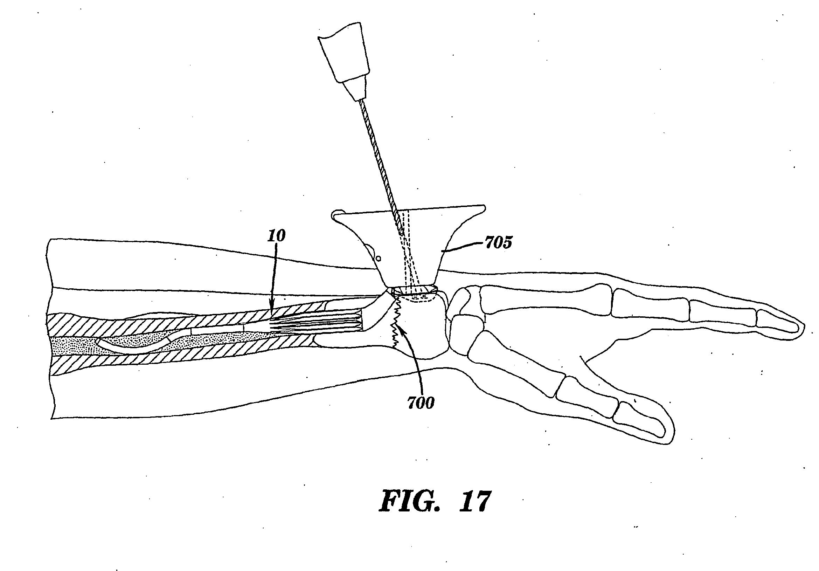

As shown in FIG. 17, following final seating of the intramedullary bone plate device 10 and while maintaining fracture reduction, a drill guide 705 may be attached to the bone plate head 100 for drilling the pilot holes through the bone screw holes 106, 107, 108, 109 in advance of inserting the bone screws 500. A depth gage is typically used (not shown) to determine the appropriate length bone screw 500 to be used when securing the bone fragments.

As shown in FIG. 18, following final tightening of the bone screws 500, the sheath 400 is placed into the sheath recess 105, allowing the nobs 402 or recesses 407 to essentially align with the opposing bone screw heads 501. The sheath 400 is then typically fixed to the bone plate head 100 with the sheath screw 600. In the event further fracture fixation is required, surgical k-wire may be inserted into the wire holes 112, 113, 114, 115 located in the medial tab member 103 with special attention being taken to ensure that the free ends of the surgical k-wire are adequately placed within the wire channels 116, 117.

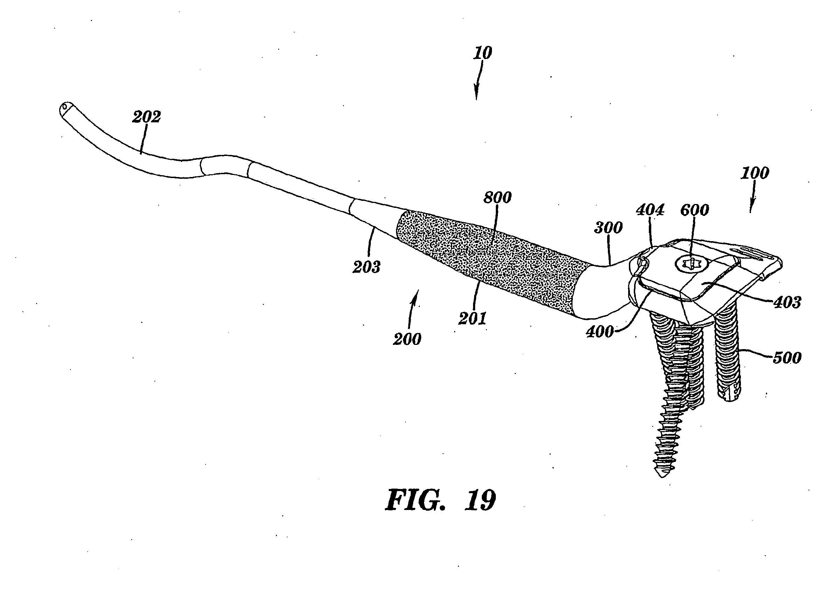

FIG. 19 shows another embodiment of the present invention, an intramedullary bone plate with sheath 10. As described previously, the distal portion 201 of the intramedullary stem 200 preferably functions to provide medullary canal fixation and rotational stability. In the alternative embodiment of the present invention, the longitudinal flutes 204 are absent from the distal portion 201. The smooth exterior surface of the distal portion 201 may be modified to enhance bone fixation and rotational stability by undergoing a surface treatment 800 that may include, but is not limited to grit blast, in-laid wire mesh and plasma spray.

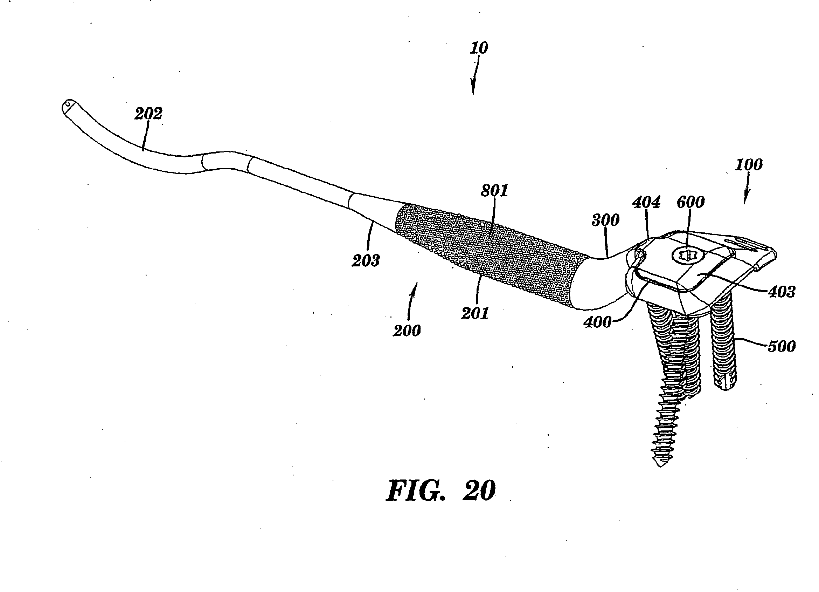

As shown in FIG. 20, a further alternative embodiment of the present invention, the smooth exterior surface of the distal portion 201 may be configured to provide enhanced bone fixation and rotational stability by the application of a three-dimension surface coating 801 that may include, but is not limited to porous-coating and bioactive agents. Such bioactive agents may include, but are not limited to tri-calcium phosphate, hydroxyapatite and bone growth factors.

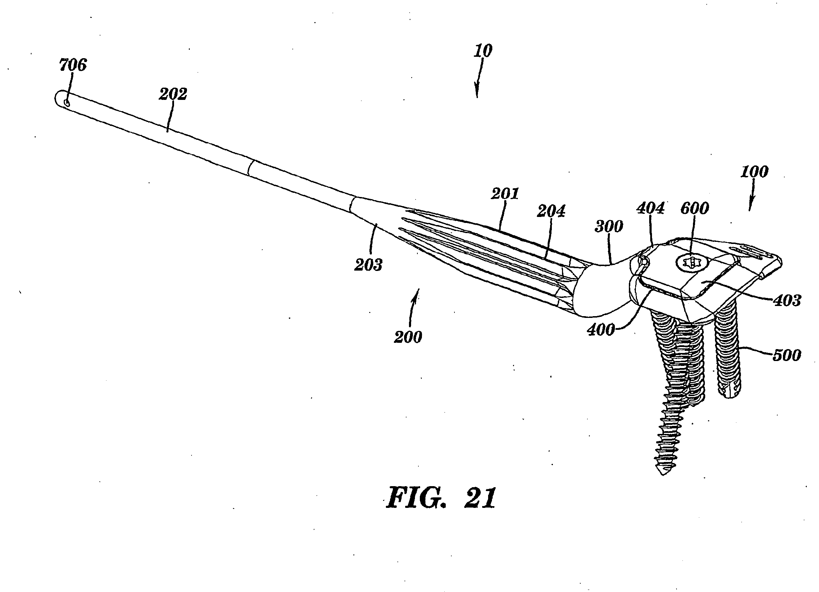

FIG. 21 illustrates yet another embodiment of the present invention, an intramedullary bone plate with sheath 10. Referencing FIG. 21 again, the proximal portion 202 of the intramedullary stem 200 is relatively straight in both the sagittal and coronal planes. A transverse through hole 706 is located along the length of the intramedullary stem. An example of this embodiment is seen in FIG. 21 wherein the transverse hole 706 is located near the tip of the intramedullary stem 200. The transverse hole 706 would allow for a pin or screw to be inserted through the cortex of the radius or other long bone in which the invention 10 is implanted. After passing through the transverse hole 706, the pin or screw may be fixed into the diametric opposite outer bone cortex, thereby substantially securing the position of the intramedullary bone plate 10.

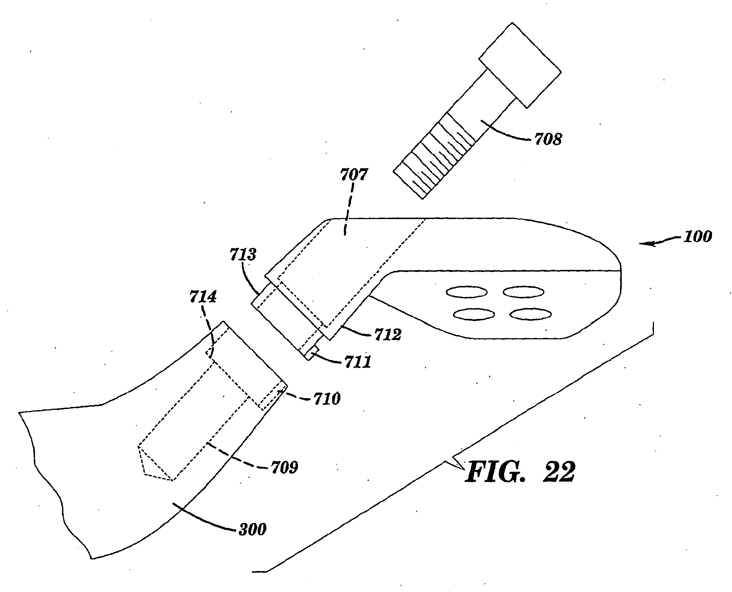

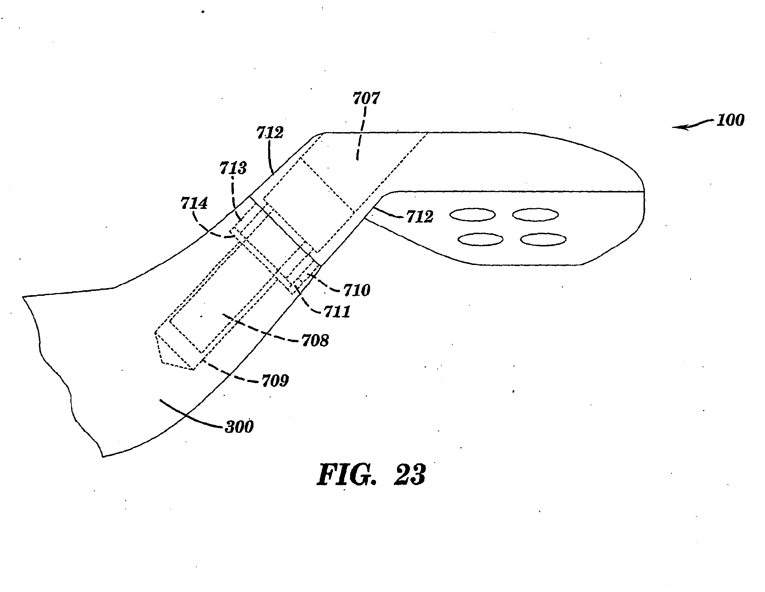

FIGS. 22 and 23 illustrates another embodiment of the present invention, an intramedullary bone plate with sheath 10. The present invention 10 provides for the intramedullary stem 200 and the bone plate head 100 to be connected in a fixed manner by a neck 300. As seen in FIGS. 22 and 23, the bone plate head 100 of the alternative embodiment is modular. On the proximal edge of the bone plate 100, a downward angled connecter 712 may be located with a through hole 707 directed along the connecter's 712 centerline. The connecter end 713 is typically of a smaller diameter relative to the connecter 712, thereby allowing the connecter end 713 to seat within the neck counter-bore 714. To substantially inhibit rotational movement of the bone plate head 100 when the connecter end 713 is inserted and seated in the neck counter-bore 714, a flange 711 may be fixed to one side of the connecter end 713. The flange 711 would typically key into a corresponding notch 710 located in the top aspect of the neck counter-bore 714. A threaded locking screw 708 may then be inserted to engage with a non-through threaded hole 709 located in the neck 300. The centerline of a threaded hole 709 being approximately concentric with the central axis of the neck 300. The preferred location of the threaded hole 709 opening being from the bottom of the neck counter-bore 714 and running to approximately the mid-shaft of the neck 300. An extended sheath (not shown) may be utilized in the alternative embodiment to cover the plurality of bone screw holes 106, 107, 108,109 and the head locking screw 708. Benefits of having a modular bone plate head 100 include intraoperative customization and inventory flexibility.

FIG. 25 illustrates another embodiment of the present invention, an intramedullary bone plate with sheath 10. This alternate embodiment may be used for treatment of fractures in bones larger than the radius. As seen in FIG. 25, the intramedullary stem 200 is relatively straight in both the sagittal and coronal planes, with the cross-section of intramedullary stem 200 being substantially circular. The angle which is formed by the neck 300 that may fix the intramedullary stem 200 to the bone plate head 100 is approximately ninety degrees relative to the intramedullary stem 200. As shown in FIG. 25, the proximal portion 202 of the intramedullary stem 200 may be tapered forming a bullet-like shaped end for insertion into the medullary canal of the fractured bone. Additionally, at least one transverse hole 706 is located along the length of the intramedullary stem 200, allowing for the insertion of a pin or screw for implant fixation purpose.

## Claims

1. An intramedullary bone plate device (10), comprising:
an intramedullary stem (200) element configured to provide fixation within a medullary canal of a bone;
a bone plate head (100) element having a top surface, a bottom surface, a lateral side and a medial side, wherein the bone plate head element (100) is comprised of a medial-lateral width which is greater or equal to one-half the proximal-distal length of said bone plate head (100) element; and
a neck (300) element fixed to an end of said intramedullary stem (200) element and extending in an upward direction and fixed to one of at least the proximal edge and bottom of said bone plate head (100) element; and
wherein said medial side comprises a downward angled and outwardly extending member (103).

2. The intramedullary bone plate device of claim 1, wherein said outwardly extending member (103) comprises at least two holes (112, 113) (114, 115) completely therethrough, and a longitudinal channel (117) (116) within the top surface of said member (103) connecting said two holes.

3. The intramedullary bone plate device of claim 1, wherein said top surface of said bone plate head element comprises a sheath recess (105) having a top surface therein.

4. The intramedullary bone plate device of claim 1, wherein said bone plate head (100) element further comprises at least one completely therethrough hole (106, 107, 108, 109).

5. The intramedullary bone plate device of claim 1 or 3, wherein said bone plate head (100) element further comprises a plurality of completely therethrough holes (106, 107, 108, 109), wherein at least one of said plurality of holes has an oblique axis relative to the others.

6. The intramedullary bone plate device of claim 3, wherein said bone plate head (100) element further comprises at least one completely therethrough hole (111) located within the sheath recess (105), with a raised collar (120) approximately concentric to said hole (111).

7. The intramedullary bone plate device of claim 3, wherein said bone plate head (100) element further comprises a plurality of completely therethrough holes (106, 107, 108, 109, 111) located within the sheath recess (105).

8. The intramedullary bone plate device of claim 7, wherein said plurality of holes:-
(a) are longitudinally and laterally (106, 107) displaced within the sheath recess;
(b) includes at least one hole (109, 111) having a centreline being about normal to the top surface of said sheath recess (105), and/or;
(c) includes at least one hole (106, 107) having a centerline being angled to the top surface of said sheath recess (105).

9. The intramedullary bone plate device of claim 7, wherein at least one of said plurality of holes:-
(a) is at a fixed angle relative to the top surface of the sheath recess (105);
(b) has a spherical concave cavity (110) relative to the top surface of the sheath recess (105), preferably wherein a bone screw (500) inserted in said holes (108) is pivotable about 0 to 10 degrees relative to the transverse plane and about 0 to 20 degrees relative to the sagittal plane, and/or;
(c) is set at a fixed angle relative to the sagittal plane and transverse plane, wherein the angular orientation of a bone screw (500) inserted in said holes (111) is rigidly fixed in the sagittal plane and transverse plane.

10. The intramedullary bone plate device of claims 1 or 8, further comprising a sheath (400) element having a top surface and bottom surface wherein said sheath element (400) has at least one hole (401) having a centerline being about normal to the top surface of said sheath (400) element; further comprising a circular groove (405) in the bottom surface of said sheath (400) element with said circular groove (405) being approximately concentric to the at least one completely therethrough hole (401).

11. The intramedullary bone plate device of claim 10, wherein the sheath element (400) has at least one of two raised nobs (402) and recesses (407) fixed to its bottom surface; said sheath element (400) being configured to attach to said bone plate element (100).

12. The intramedullary bone plate device of claims 1, 3, 5 and 10:-
(a) wherein said sheath element (400) is configured to be fixed within the sheath recess (105), wherein said sheath element (400) is fixed within the sheath recess (105) with a threaded screw (500);
(b) wherein said sheath element (400) covers said plurality of holes, and/or;
(c) further comprising means for substantially inhibiting any movement of an inserted bone screw (500), wherein said means comprises a sheath element (400) fixed to a sheath recess (407).

13. The intramedullary bone plate device of claim 1, wherein said intramedullary stem (200) element further comprises a distal portion, a mid-shaft portion and a proximal portion.

14. The intramedullary bone plate device of claim 1, wherein said intramedullary stem element (200) is:-
(a) longitudinally displaced from said bone plate head (100) element, and wherein said neck element (300) connects the end of the distal portion (201) of said intramedullary stem (200) element to at least one of the edge and bottom of said bone plate (100) head element forming an angle of ninety degrees or less;
(b) straight in the coronal plane, said bone plate head (100) element has a convex top surface and concave bottom surface relative to the transverse plane, and/or;
(c) substantially circular in cross-section.

15. The intramedullary bone plate device of claim 13, wherein:-
(a) the distal portion (201) and mid-shaft portion (203) of said intramedullary stem (200) element is straight in the sagittal plane and the proximal portion of said intramedullary stem element (200) is curved in the sagittal plane;
(b) the diameter of the proximal end of the distal portion tapers in the mid-shaft portion (203) until said diameter matches the smaller diameter of the proximal portion (202) of said intramedullary stem (200) element, and/or;
(c) said intramedullary stem (200) element is configured to provide fixation within a medullary canal of a bone, wherein the configuration is comprised of full circumference, longitudinal flutes (204) extending from and including the distal portion (201) to the mid-shaft portion (203).

## Patentansprüche

1. Eine intramedulläre Knochenplattenvorrichtung (10), die Folgendes beinhaltet:
ein intramedulläres Stammelement (200), das konfiguriert ist, um innerhalb eines Markraums eines Knochens eine Befestigung bereitzustellen;
ein Knochenplattenkopfelement (100) mit einer oberen Oberfläche, einer unteren Oberfläche, einer lateralen Seite und einer medialen Seite, wobei das Knochenplattenkopfelement (100) eine medial-laterale Breite beinhaltet, die größer oder gleich einer Hälfte der proximaldistalen Länge des Knochenplattenkopfelements (100) ist; und
ein Halselement (300), das an einem Ende des intramedullären Stammelements (200) befestigt ist und sich in einer Richtung nach oben erstreckt und an mindestens einem von der proximalen Kante und dem Unterteil des Knochenplattenkopfelements (100) befestigt ist, und
wobei die mediale Seite einen nach unten abgewinkelten und sich nach außen erstreckenden Teil (103) beinhaltet.

2. Intramedulläre Knochenplattenvorrichtung gemäß Anspruch 1, wobei der sich nach außen erstreckende Teil (103) mindestens zwei komplett **dadurch** verlaufende Löcher (112, 113) (114, 115) und innerhalb der oberen Oberfläche des Teils (103) einen longitudinalen Kanal (117) (116), der die zwei Löcher verbindet, beinhaltet.

3. Intramedulläre Knochenplattenvorrichtung gemäß Anspruch 1, wobei die obere Oberfläche des Knochenplattenkopfelements eine Umhüllungsaussparung (105) mit einer oberen Oberfläche darin beinhaltet.

4. Intramedulläre Knochenplattenvorrichtung gemäß Anspruch 1, wobei das Knochenplattenkopfelement (100) ferner mindestens ein Komplettdurchgangsloch (106, 107, 108, 109) beinhaltet.

5. Intramedulläre Knochenplattenvorrichtung gemäß Anspruch 1 oder 3, wobei das Knochenplattenkopfelement (100) ferner eine Vielzahl von Komplettdurchgangslöchern (106, 107, 108, 109) beinhaltet, wobei mindestens eins der Vielzahl von Löchern eine schräge Achse relativ zu den anderen aufweist.

6. Intramedulläre Knochenplattenvorrichtung gemäß Anspruch 3, wobei das Knochenplattenkopfelement (100) ferner mindestens ein Komplettdurchgangsloch (111), das sich innerhalb der Umhüllungsaussparung (105) befindet, mit einem erhöhten Kragen (120) etwa konzentrisch zu dem Loch (111) beinhaltet.

7. Intramedulläre Knochenplattenvorrichtung gemäß Anspruch 3, wobei das Knochenplattenkopfelement (100) ferner eine Vielzahl von Komplettdurchgangslöchern (106, 107, 108, 109, 111), die sich innerhalb der Umhüllungsaussparung (105) befinden, beinhaltet.

8. Intramedulläre Knochenplattenvorrichtung gemäß Anspruch 7, wobei die Vielzahl der Löcher
(a) innerhalb der Umhüllungsaussparung longitudinal und lateral (106, 107) versetzt ist;
(b) mindestens ein Loch (109, 111) mit einer Mittellinie, die zu der oberen Oberfläche der Umhüllungsaussparung (105) etwa senkrecht ist, umfasst, und/oder
(c) mindestens ein Loch (106, 107) mit einer Mittellinie, die zu der oberen Oberfläche der Umhüllungsaussparung (105) abgewinkelt ist, umfasst.

9. Intramedulläre Knochenplattenvorrichtung gemäß Anspruch 7, wobei mindestens eins der Vielzahl von Löchern
(a) in einem festen Winkel relativ zu der oberen Oberfläche der Umhüllungsaussparung (105) befindlich ist;
(b) einen sphärischen konkaven Hohlraum (110) relativ zu der oberen Oberfläche der Umhüllungsaussparung (105) aufweist, wobei vorzugsweise eine Knochenschraube (500), die in die Löcher (108) eingeführt ist, um 0 bis 10 Grad relativ zu der Transversalebene und um 0 bis 20 Grad relativ zu der Sagittalebene schwenkbar ist, und/oder
(c) in einem festen Winkel relativ zu der Sagittalebene und der Transversalebene eingerichtet ist, wobei die Winkelausrichtung einer Knochenschraube (500), die in die Löcher (111) eingeführt ist, in der Sagittalebene und der Transversalebene starr befestigt ist.

10. Intramedulläre Knochenplattenvorrichtung gemäß Anspruch 1 oder 8, die ferner ein Umhüllungselement (400) mit einer oberen Oberfläche und einer unteren Oberfläche beinhaltet, wobei das Umhüllungselement (400) mindestens ein Loch (401) mit einer Mittellinie, die zu der oberen Oberfläche des Umhüllungselements (400) etwa senkrecht ist, aufweist; die in der unteren Oberfläche des Umhüllungselements (400) ferner eine kreisförmige Rille (405) beinhaltet, wobei die kreisförmige Rille (405) zu dem mindestens einen Komplettdurchgangsloch (401) etwa konzentrisch ist.

11. Intramedulläre Knochenplattenvorrichtung gemäß Anspruch 10, wobei das Umhüllungselement (400) mindestens eins von zwei erhöhten Vorsprüngen (402) und Aussparungen (407), die an seiner unteren Oberfläche befestigt sind, aufweist; wobei das Umhüllungselement (400) konfiguriert ist, um an dem Knochenplattenelement (100) angebracht zu werden.

12. Intramedulläre Knochenplattenvorrichtung gemäß Anspruch 1, 3, 5 und 10,
(a) wobei das Umhüllungselement (400) konfiguriert ist, um innerhalb der Umhüllungsaussparung (105) befestigt zu werden, wobei das Umhüllungselement (400) mit einer Gewindeschraube (500) innerhalb der Umhüllungsaussparung (105) befestigt ist;
(b) wobei das Umhüllungselement (400) die Vielzahl von Löchern abdeckt und/oder
(c) die ferner ein Mittel im Wesentlichen zum Unterbinden jeder Bewegung einer eingeführten Knochenschraube (500) beinhaltet, wobei das Mittel ein Umhüllungselement (400), das an einer Umhüllungsaussparung (407) befestigt ist, beinhaltet.

13. Intramedulläre Knochenplattenvorrichtung gemäß Anspruch 1, wobei das intramedulläre Stammelement (200) ferner einen distalen Abschnitt, einen Mittelschaftabschnitt und einen proximalen Abschnitt beinhaltet.

14. Intramedulläre Knochenplattenvorrichtung gemäß Anspruch 1, wobei das intramedulläre Stammelement (200)
(a) von dem Knochenplattenkopfelement (100) longitudinal versetzt ist und wobei das Halselement (300) das Ende des distalen Abschnitts (201) des intramedullären Stammelements (200) mit mindestens einem von der Kante und dem Unterteil des Knochenplattenkopfelements (100) verbindet, wobei ein Winkel von neunzig Grad oder weniger gebildet wird;
(b) in der Koronalebene gerade ist, wobei das Knochenplattenkopfelement (100) eine konvexe obere Oberfläche und eine konkave untere Oberfläche relativ zu der Transversalebene aufweist und/oder
(c) im Querschnitt im Wesentlichen kreisförmig ist.

15. Intramedulläre Knochenplattenvorrichtung gemäß Anspruch 13, wobei
(a) der distale Abschnitt (201) und der Mittelschaftabschnitt (203) des intramedullären Stammelements (200) in der Sagittalebene gerade sind und der proximale Abschnitt des intramedullären Stammelements (200) in der Sagittalebene gekrümmt ist;
(b) sich der Durchmesser des proximalen Endes des distalen Abschnitts in dem Mittelschaftabschnitt (203) verjüngt, bis der Durchmesser mit dem kleineren Durchmesser des proximalen Abschnitts (202) des intramedullären Stammelements (200) übereinstimmt, und/oder
(c) das intramedulläre Stammelement (200) konfiguriert ist, um innerhalb eines Markraums eines Knochens eine Befestigung bereitzustellen, wobei die Konfiguration vollumlaufende, longitudinale Nuten (204), die sich von dem distalen Abschnitt (201) zu dem Mittelschaftabschnitt (203) erstrecken und den distalen Abschnitt einschließen, beinhaltet.

## Revendications

1. Un dispositif formant plaque osseuse intramédullaire (10), comprenant :
un élément formant tige intramédullaire (200) configuré pour fournir une fixation au sein d'un canal médullaire d'un os ;
un élément formant tête de plaque osseuse (100) ayant une surface de dessus, une surface de dessous, un côté latéral et un côté médial, où l'élément formant tête de plaque osseuse (100) se compose d'une largeur médio-latérale qui est supérieure ou égale à la moitié de la longueur proximo-distale dudit élément formant tête de plaque osseuse (100) ; et
un élément formant col (300) fixé sur une extrémité de l'élément formant tige intramédullaire (200) et s'étendant dans une direction vers le haut et fixé au moins soit sur le bord proximal, soit sur le dessous dudit élément formant tête de plaque osseuse (100) ; et
où ledit côté médial comprend un organe en angle vers le bas et s'étendant vers l'extérieur (103).

2. Le dispositif formant plaque osseuse intramédullaire de la revendication 1, où ledit organe s'étendant vers l'extérieur (103) comprend au moins deux trous (112, 113) (114, 115) le traversant complètement, et un canal longitudinal (117) (116) au sein de la surface de dessus dudit organe (103) raccordant lesdits deux trous.

3. Le dispositif formant plaque osseuse intramédullaire de la revendication 1, où ladite surface de dessus dudit élément formant tête de plaque osseuse comprend un renfoncement pour gaine (105) ayant une surface de dessus dans celui-ci.

4. Le dispositif formant plaque osseuse intramédullaire de la revendication 1, où ledit élément formant tête de plaque osseuse (100) comprend en outre au moins un trou le traversant complètement (106, 107, 108, 109).

5. Le dispositif formant plaque osseuse intramédullaire de la revendication 1 ou 3, où ledit élément formant tête de plaque osseuse (100) comprend en outre une pluralité de trous le traversant complètement (106, 107, 108, 109), où au moins un trou parmi la pluralité de trous a un axe oblique par rapport aux autres.

6. Le dispositif formant plaque osseuse intramédullaire de la revendication 3, où ledit élément formant tête de plaque osseuse (100) comprend en outre au moins un trou le traversant complètement (111) situé au sein du renfoncement pour gaine (105), avec un collier surélevé (120) approximativement concentrique audit trou (111).

7. Le dispositif formant plaque osseuse intramédullaire de la revendication 3, où ledit élément formant tête de plaque osseuse (100) comprend en outre une pluralité de trous le traversant complètement (106, 107, 108, 109, 111) situés au sein du renfoncement pour gaine (105).

8. Le dispositif formant plaque osseuse intramédullaire de la revendication 7, où ladite pluralité de trous :
(a) sont déplacés de façon longitudinale et latérale (106, 107) au sein du renfoncement pour gaine ;
(b) incluent au moins un trou (109, 111) ayant une ligne centrale environ perpendiculaire à la surface de dessus dudit renfoncement pour gaine (105), et / ou ;
(c) incluent au moins un trou (106, 107) ayant une ligne centrale en angle par rapport à la surface de dessus dudit renfoncement pour gaine (105).

9. Le dispositif formant plaque osseuse intramédullaire de la revendication 7, où au moins un trou parmi ladite pluralité de trous :
(a) se trouve à un angle fixe par rapport à la surface de dessus du renfoncement pour gaine (105) ;
(b) a une cavité concave sphérique (110) par rapport à la surface de dessus du renfoncement pour gaine (105), de préférence où une vis à os (500) insérée dans lesdits trous (108) peut pivoter d'environ 0 à 10 degrés par rapport au plan transversal et d'environ 0 à 20 degrés par rapport au plan sagittal, et / ou ;
(c) est établi à un angle fixe par rapport au plan sagittal et au plan transversal, où l'orientation angulaire d'une vis à os (500) insérée dans lesdits trous (111) est fixe de façon rigide dans le plan sagittal et le plan transversal.

10. Le dispositif formant plaque osseuse intramédullaire des revendications 1 ou 8, comprenant en outre un élément formant gaine (400) ayant une surface de dessus et une surface de dessous où ledit élément formant gaine (400) a au moins un trou (401) ayant une ligne centrale environ perpendiculaire à la surface de dessus dudit élément formant gaine (400) ; comprenant en outre une rainure circulaire (405) dans la surface de dessous dudit élément formant gaine (400), ladite rainure circulaire (405) étant approximativement concentrique à cet au moins un trou le traversant complètement (401).

11. Le dispositif formant plaque osseuse intramédullaire de la revendication 10, où l'élément formant gaine (400) a au moins soit deux projections surélevées (402), soit deux renfoncements (407) fixés sur sa surface de dessous ; ledit élément formant gaine (400) étant configuré pour être rattaché audit élément formant plaque osseuse (100).

12. Le dispositif formant plaque osseuse intramédullaire des revendications 1, 3, 5 et 10 :
(a) où ledit élément formant gaine (400) est configuré pour être fixé au sein du renfoncement de gaine (105), où ledit élément formant gaine (400) est fixé au sein du renfoncement pour gaine (105) à l'aide d'une vis filetée (500) ;
(b) où ledit élément formant gaine (400) recouvre ladite pluralité de trous, et 1 ou ;
(c) comprenant en outre un moyen destiné à empêcher substantiellement tout déplacement d'une vis à os insérée (500), où ledit moyen comprend un élément formant gaine (400) fixé sur un renfoncement pour gaine (407).

13. Le dispositif formant plaque osseuse intramédullaire de la revendication 1, où ledit élément formant tige intramédullaire (200) comprend en outre une portion distale, une portion à mi-arbre et une portion proximale.

14. Le dispositif formant plaque osseuse intramédullaire de la revendication 1, où ledit élément formant tige intramédullaire (200) est :
(a) déplacé de façon longitudinale depuis ledit élément formant tête de plaque osseuse (100), et où ledit élément formant col (300) raccorde l'extrémité de la portion distale (201) dudit élément formant tige intramédullaire (200) au moins soit au bord, soit au dessous dudit élément formant tête de plaque osseuse (100) formant un angle de quatre-vingt-dix degrés ou moins ;
(b) droit dans le plan frontal, ledit élément formant tête de plaque osseuse (100) a une surface de dessus convexe et une surface de dessous concave par rapport au plan transversal, et / ou ;
(c) substantiellement circulaire en coupe transversale.

15. Le dispositif formant plaque osseuse intramédullaire de la revendication 13, où :
(a) la portion distale (201) et la portion à mi-arbre (203) dudit élément formant tige intramédullaire (200) sont droites dans le plan sagittal et la portion proximale dudit élément formant tige intramédullaire (200) est incurvée dans le plan sagittal ;
(b) le diamètre de l'extrémité proximale de la portion distale s'effile dans la portion à mi-arbre (203) jusqu'à ce que ledit diamètre corresponde au diamètre plus petit de la portion proximale (202) dudit élément formant tige intramédullaire (200), et / ou ;
(c) ledit élément formant tige intramédullaire (200) est configuré pour fournir une fixation au sein d'un canal médullaire d'un os, où la configuration se compose de cannelures longitudinales de circonférence complète (204) s'étendant de la portion distale (201), et incluant celle-ci, à la portion à mi-arbre (203).
